# EUROPÄISCHE PATENTSCHRIFT

(11) **EP 2 405 960 B1**
(45) Veröffentlichungstag und Bekanntmachung des Hinweises auf die Patenterteilung: **19.04.2017**
(21) Anmeldenummer: 10707474.2
(22) Anmeldetag: 04.03.2010
(51) Int. Cl.: A61M 5/145

(54) **VORRICHTUNG ZUM KONTINUIERLICHEN ANTRIEB EINES SPRITZENKOLBENS MIT VERMINDERTEM HAFT-GLEIT-EFFEKT**
DRIVE FOR GENERATING CONTINUOUS MOVEMENT OF A SYRINGE PISTON WITH REDUCED STICK-SLIP EFFECT
PROCÉDÉ ET DISPOSITIF D'ENTRAÎNEMENT CONTINU D'UN PISTON DE SERINGUE

(30) Priorität: 12.03.2009 DE 102009012449
(43) Veröffentlichungstag der Anmeldung: 18.01.2012
(73) Patentinhaber: Imp Pape GmbH & Co. Kg, 30900 Wedemark (DE); Licher Medizintechnologie GmbH, 30900 Wedemark (DE)
(72) Erfinder: MUSHOFF, Dieter, 30900 Wedemark (DE); PAPE, Manfred, 31249 Hohenhameln (DE)
(74) Vertreter: Lins, Martina
(86) Internationale Anmeldenummer: PCT/EP2010/001348
(87) Internationale Veröffentlichungsnummer: WO 2010/102750

(56) Entgegenhaltungen:
- EP-A1- 1 752 172
- WO-A1-00/25844
- WO-A2-2007/075677
- GB-A- 1 528 385
- US-A1- 2002 007 139
- US-B1- 6 423 035

## Beschreibung

Die Erfindung betrifft eine Vorrichtung zum kontinuierlichen und hochpräzisen Antrieb eines Kolbens in einer medizinischen Infusions- oder Injektionsspritze.

Derartige Vorrichtungen sind grundsätzlich bekannt und werden auch als "Spritzenpumpen" bezeichnet. Bei der Erfindung wird ein neues Verfahren zur Förderung des Spritzenkolbens mit Hilfe einer Spritzenpumpe verwirklicht.

Unter "Spritzenpumpe" versteht der Fachmann ein Gerät, das den Kolben einer in das Gerät eingespannten Infusions- oder Injektionsspritze so vorwärts bewegt, dass eine zu applizierende Medikamentendosis oder eine Injektionslösung in den Körper eines Patienten gepumpt, d. h. gefördert werden kann.

In der medizinischen Infusionstechnik ist es heute in bestimmten Fällen erforderlich, Medikamente sehr langsam übe einen Zeitraum von vielen Stunden oder Tagen zuzuführen. Therapieformen, bei denen dies erforderlich ist, umfassen bestimmte Chemotherapien, Schmerztherapien, Gentherapien, Antikörpertherapien, spinale Applikationen und anderes mehr. Ein Beispiel ist die Zuführung von Apomorphin (einem Dopamin-Agonisten) bei Morbus Parkinson. Hierfür ist eine absolut kontinuierliche dopaminerge Stimulation erforderlich.

Im Stand der Technik wird der Linearantrieb für den Spritzenkolben derzeit mit Hilfe von Schrittmotoren realisiert, da anders die sehr langsame Förderung nicht möglich ist. Diese Schrittmotoren bewegen den Kolben in sehr kleinen Schritten voran. Die Schritt-Taktung kann beliebig langsam sein, die Förderung der Infusionslösung bzw. des Medikaments erfolgt dadurch jedoch prinzipiell pulsatil.

Auch bei kontinuierlicher, analog gesteuerter Vorwärtsbewegung des Kolbens kommt es zu einer schubweisen Bewegung in Folge so genannter "slip-stick"-Effekte. Dabei gewinnt die Haftreibung der Dichtung des Kolbens am Spitzenzylinder gegenüber der Gleitreibung bei extrem langsamem Vorschub die Überhand und führt zu einem Stocken der Vorwärtsbewegung. Der sich aufbauende Förderdruck am Kolben überwindet die Haftreibung. Nach der Anfangsbeschleunigung kommt es wieder zum Stocken aufgrund der sehr langsamen Bewegung. Eine pulsatile Fördercharakteristik ist dabei physikalisch unvermeidlich.

Letztlich ist daher im Stand der Technik eine hundertprozentig konstante Förderung nicht möglich. Dadurch können therapeutische Bereiche und deren Ober- und Untergrenzen überschritten werden. Für bestimmte Therapien, wie oben genannt, sind jegliche Konzentrationsschwankungen im Plasma, pulsatil oder sinusförmig, unerwünscht. Bei intraspinalen Applikationen beispielsweise verhindern durch "slip-stick" verursachte Druckspitzen die Einhaltung der vorgegebenen Volumina und können zu Gewebeschäden im neuronalen Gewebe führen.

Die US 2002/007139 A1 beschreibt eine Vorrichtung zur Abgabe einer Flüssigkeit, bei der ein Behältereinsatz in eine Aufnahmeeinrichtung aufgenommen ist. Bei Drehung der Aufnahmeeinrichtung wird der Behältereinsatz translatorisch auf den verschiebungsstarren Kolben zubewegt. In einer weiteren Ausgestaltung verfährt der Kolben der Dosiervorrichtung translatorisch sowie rotatorisch durch den Behältereinsatz. Zur Vermeidung von "slip-stick"-Effekten ist der Behältereinsatz in einer speziellen Art und Weise aufgebaut, beispielsweise weist der Behältereinsatz in der ersten Ausgestaltung ein Außengewinde auf. Der Aufbau ist erkennbar für größere Volumenströme ausgelegt. Kleinste Förderraten sind damit nicht realisierbar.

Der Erfindung liegt daher die Aufgabe zugrunde, mit Hilfe einer neu zu entwickelnden Spritzenpumpe einen absolut kontinuierlich und hochpräzise arbeitenden Kolbenantrieb zu verwirklichen.

Die Aufgabe wird gelöst durch eine Vorrichtung gemäß Anspruch 1. Diese Vorrichtung ermöglicht ein Verfahren, bei welchem der Kolben einer Infusions- oder Injektionsspritze kontinuierlich motorisch angetrieben wird und der translatorischen Vortriebsbewegung des Kolbens eine rotatorische Bewegung überlagert wird, wobei die (schraubende) Bewegung der kontinuierlich durch den Motor angetriebenen Bewegungsschraube, mit oder ohne Untersetzung, auf den Kolben der Spritze übertragen wird. Der vollständig kontinuierliche Antrieb wird vorzugsweise durch eine Analogsteuerung umgesetzt, weiter vorzugsweise wird ein kontinuierlich in seiner Leistung regelbarer Gleichstrommotor verwendet.

Der Kolben führt während der Förderbewegung, mit der die Infusions- oder Arzneimittellösung bzw. das Medikament in den Körper eines Patienten gepumpt wird, eine schraubende Bewegung aus, so dass im Verhältnis zur Vorwärtsbewegung des Kolbens zusätzlich eine - vorzugsweise von der zurückgelegten Wegstrecke her größere - Bewegung in Umfangsrichtung erfolgt. Die Wegstrecke, die ein Berührungspunkt zwischen Spritzenkörper und Kolben bei der Vorwärts- bzw. Förderbewegung zurücklegt, vergrößert sich also umsomehr, je flacher die Steigung der schraubenden Bewegung ist, d.h. je größer der Anteil der Rotation im Verhältnis zur Translation ist.

Das Verfahren wird so ausgeführt, dass für die gleichzeitige Erzeugung von Rotations- und Vorwärtsbewegung eine Bewegungsschraube verwendet wird, die mit einer passenden Mutter zusammenwirkt.

Eine Bewegungsschraube - auch als Spindel oder Gewindespindel bezeichnet - setzt üblicherweise eine Rotationsbewegung in eine Translationsbewegung relativ zu einer feststehenden Mutter um. Dabei wird die Spindel in der Regel so angekuppelt, dass die Drehbewegung gerade nicht auf den Vorschubgeber oder das zu bewegende Teil übertragen wird.

Bei der Erfindung wird sowohl die Rotationsbewegung als auch die Translationsbewegung, d.h. der Vorschub der Bewegungsschraube für die Fortbewegung des Kolbens im Spritzenkörper genutzt.

Das Losreißmoment aus der Haftreibung wirkt deshalb hauptsächlich in Umfangsrichtung, so dass ein Anrucken (slip-stick) für die Geradeausbewegung vernachlässigt werden kann.

Durch die Kombination der Drehbewegung in Überlagerung zur Schubbewegung wird ein slip-stick-Effekt in der für die Förderung maßgeblichen Schubbewegung verhindert, so dass der Kolben zu jedem Augenblick seiner Bewegung in Förderrichtung in Gleitreibung bleibt.

Hierdurch werden hochpräzise Mengenförderraten ermöglicht und Konzentrationsspitzen bei der Medikamentendosierung vermieden, was bestimmte Therapien erst möglich macht.

Die Erfindung ermöglicht die Verabreichung des Spritzeninhalts mit einer regelbaren, vollständig kontinuierlichen, sehr gleichmäßigen Förderleistung. Die durch den Antrieb vorgegebene Leistung wird stufenlos umgesetzt und gegebenenfalls zwischen 100 % und nach Null untersetzt.

Bei der erfindungsgemäßen Vorrichtung zum Antreiben eines Kolbens einer Infusions- oder Injektionsspritze mit Hilfe einer Spritzenpumpe, in die die Infusions- oder Injektionsspritze eingesetzt oder eingespannt ist, wird die oben angegebene Aufgabe dadurch gelöst, dass mit dem Kolben der Spritze - unmittelbar oder über einen Adapter - eine Bewegungsschraube als Druckgeber für den Kolben verbunden ist, wobei die schraubende Bewegung der Bewegungsschraube (auch Spindel genannt), während sich die Bewegungsschraube durch die zugehörige Mutter bewegt, auf den Kolben übertragen wird, und dass die Bewegungsschraube mit der Welle eines kontinuierlich arbeitenden Motors direkt oder durch einen Überträger (1:1) oder Untersetzer (mit Über- oder Untersetzung) verbunden ist und eine Mutter der Bewegungsschraube an einem Gehäuseteil der Vorrichtung befestigt ist.

Die erfindungsgemäße Vorrichtung wird besonders häufig für die sehr langsame Zufuhr von Medikamenten/Infusionen/Injektionen benötigt. Der Kolbenvorschub muss demnach ebenfalls langsam sein. Entsprechend werden in der Regel stark untersetzende Drehzahlwandler für die Motoruntersetzung zum Einsatz kommen.

Grundsätzlich ist die erfindungsgemäße Vorrichtung jedoch auch für eine schnelle, gleichmäßige Medikamentenverabreichung geeignet.

Bei der Erfindung wird also, wie beschrieben, der Antrieb des Kolbens, bzw. der Vortrieb des Kolbens während des Pumpvorgangs, dadurch bewerkstelligt, dass als Druckgeber eine kontinuierlich durch einen Motor angetriebene Gewindespindel verwendet wird.

Das Gewinde der Spindel ist der geforderten Förderrate angepasst.

Weiter vorzugsweise ist an der Vorrichtung auch eine Einspannvorrichtung für die Spritze vorhanden. Grundsätzlich kann die Spritze allerdings auch unabhängig von der Pumpenspritze in räumlicher Zuordnung mit einer gesonderten Einspannvorrichtung eingespannt werden, oder sie kann beispielsweise in das Gehäuse der Spritzenpumpe, vorzugsweise quer zur Kolbenbewegungsrichtung, so eingesetzt werden, dass sie in Bewegungsrichtung der Bewegungsspindel und des Kolbens fixiert ist

Bevorzugt ist vorgesehen, dass die Gewindespindel oder Bewegungsschraube formschlüssig mit dem Kolben oder einem mit dem Kolben verbundenen Adapter verbunden, insbesondere verschraubt ist.

Gemäß einer Ausführungsform der Erfindung wird der Motor auf einem Schlitten der Spindel nachgeführt. Hierfür bewegt sich der Schlitten mit dem Motor beispielsweise auf einer Gleitschiene, während sich die Bewegungsschraube mit dem Kolben relativ zur ortsfesten Mutter vorwärts bewegt.

Gemäß einer weiteren bevorzugten Ausführungsform ist der Motor ortsfest angeordnet und die Motorwelle ist mit einer Mitnehmergabel verbunden, die an einem an der Bewegungsschraube befestigten Mitnehmerstift angreift. Die Mitnehmergabel macht ausschließlich die Rotationsbewegung des ortsfesten Motors mit, während die Bewegungsschraube sich mit dem Kolben relativ zur ortsfesten Mutter vorwärts bewegt, also die aus der Rotation umgesetzte Translation ausführt.

Die Mitnehmergabel kann vorzugsweise in Form einer Hülse mit wenigstens einer schlitzförmigen Öffnung ausgebildet sein. In der schlitzförmigen Öffnung gleitet der Mitnehmerstift während der Vorwärtsbewegung der Spindel/Bewegungsschraube nach vorne.

Im Folgenden wird die Erfindung anhand von Beispielen näher erläutert, die in der Zeichnung dargestellt sind. Die Beispiele dienen ausschließlich zur Illustration und zum leichteren Verständnis der Erfindung in Bezug auf praktische Beispiele, sollen jedoch keinesfalls beschränkend ausgelegt werden. Der Fachmann wird ohne Weiteres in der Lage sein, im Rahmen des oben Beschriebenen weitere Beispiele zu konstruieren.

In der Zeichnung zeigen (nicht maßstabsgetreu):
- Fig. 1: ein erstes Ausführungsbeispiel für eine Spritzenpumpe einschließlich Spritze, schematisch im Längsschnitt dargestellt,
- Fig. 2a: ein zweites Ausführungsbeispiel für eine Spritzenpumpe einschließlich Spritze, schematisch im Längsschnitt dargestellt,
- Fig. 2b: eine schematischen Teilansicht (Draufsicht) für das Ausführungsbeispiel aus Figur 2a, gesehen in Richtung des Pfeils "A" aus Fig. 2a.
- Fig. 3a: ein drittes Ausführungsbeispiel für eine Spritzenpumpe einschließlich Spritze, schematisch im Längsschnitt dargestellt,
- Fig. 3b: eine schematischen Teilansicht (Draufsicht) für das Ausführungsbeispiel aus Figur 3a, gesehen in Richtung des Pfeils "A" aus Fig. 3a.

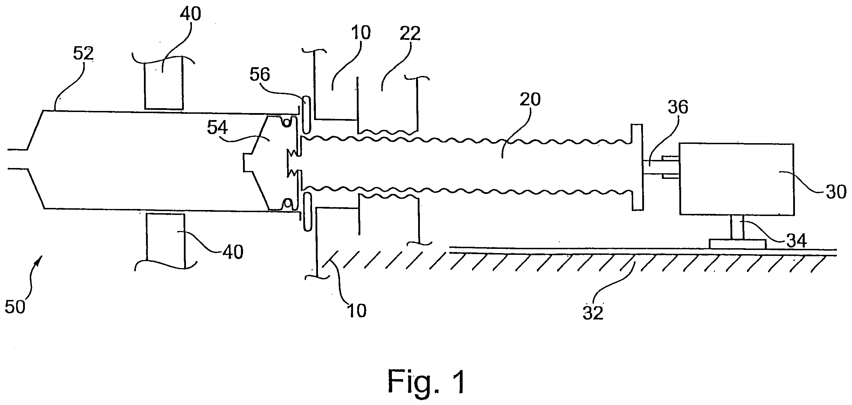

Figur 1 zeigt den schematischen Längsschnitt einer Vorrichtung mit einer Spritzenpumpe mit einem Gehäuse 10, einer Bewegungsschraube 20 und einem Motor 30. Die Mutter 22 der Bewegungsschraube 20 ist fest mit dem Gehäuse 10 verbunden, beide sind ortsfest angeordnet. Ferner ist mit dem Gehäuse 10 eine Gleitschiene 32 verbunden, auf der ein Schlitten 34 gleitet, mit Hilfe dessen der Motor 30 der Bewegungsschraube 20 Kraft seines eigenen Antriebs nachgeführt wird. Die Motorwelle 36 ist mit der Bewegungsschraube 20 form- oder kraftschlüssig fest verbunden, so dass sich die Drehbewegung des Motors 30 in diesem Beispiel 1:1 auf die Bewegungsschraube 20 überträgt. Die Vorrichtung umfasst weiter eine Einspannvorrichtung 40 für eine im Ganzen mit 50 bezeichnete Spritze. Die Einspannvorrichtung 40 fixiert die Spritze 50 vor dem Gehäuse 10 der Spritzenpumpe und ist (hier nicht dargestellt) mit diesem vorzugsweise fest verbunden. Die Spritze besitzt einen Spritzenkörper 52, einen Kolben 54 und einen Kolbenstopp 56. Die Bewegungsschraube 20 und der Kolben 54 sind verschraubt und auf diese Weise formschlüssig verbunden. Die Bewegungsschraube 20 wirkt daher unmittelbar als Druckgeber auf den Kolben 54 der eingespannten Spritze 50. Die Drehbewegung des Motors 30 wird in eine vollständig gleichmäßige, nicht schrittweise oder pulsatile Translation umgesetzt. Durch die überlagerte Drehbewegung können Slip-Stick-Effekte vernachlässigt werden. Ein im Spritzenkörper 52 befindliches Arzneimittel (in Form einer Lösung oder Suspension) wird gleichmäßig, also in hochpräziser Weise kontinuierlich ausgepresst.

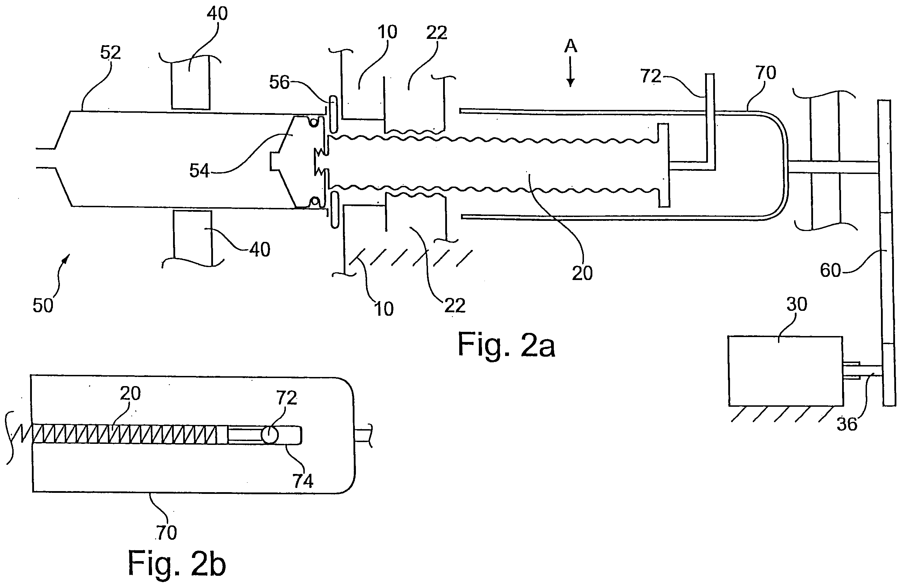

Figur 2 zeigt ein weiteres Ausführungsbeispiel für eine erfindungsgemäße Vorrichtung. Sich entsprechende Teile sind mit gleichen Bezugsziffern gekennzeichnet.

Figur 2a zeigt eine Spritzenpumpe, bei der das Gehäuse 10, die Bewegungsschraube 20, der Motor 30 und die Einspannvorrichtung 40 für die Spritze 50 ortsfest angeordnet sind. Die Drehbewegung des Motors 30 wird in diesem Beispiel durch eine Übertragung 60 über Riemen auf eine Mitnehmergabel 70 übertragen, die mit einem Mitnehmerstift 72 zusammenwirkt. Dabei gleitet, wie in Figur 2b zu erkennen, der Mitnehmerstift 72 in einem Schlitz 74 der Mitnehmergabel 70, während sich die Bewegungsschraube 20 durch die Mutter 22 vorwärts bewegt und dabei den Mitnehmerstift 72 mitnimmt.

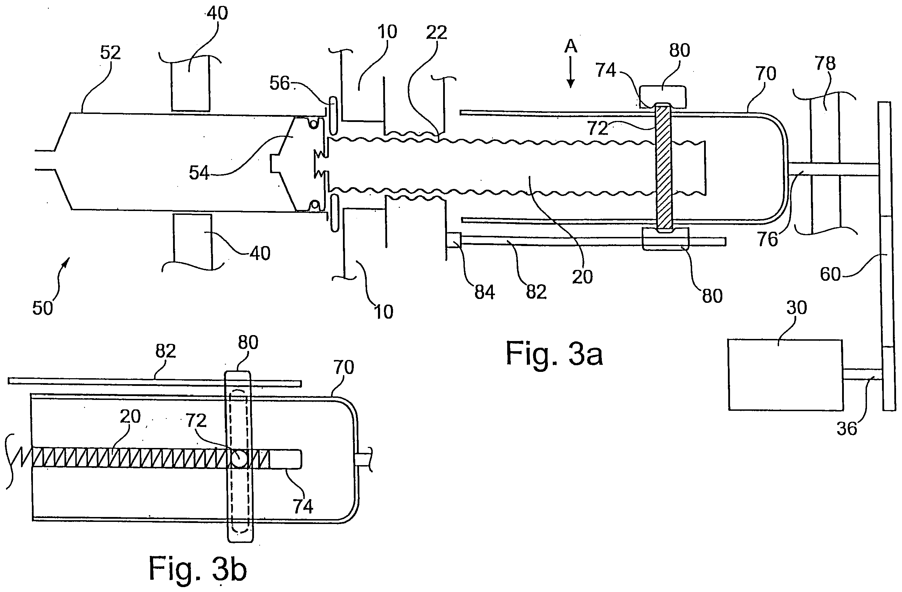

Figur 3a zeigt ein weiteres Ausführungsbeispiel der erfindungsgemäßen Vorrichtung (Spritzenpumpe, ggf. kombiniert mit Einspannvorrichtung). Gleiche Teile sind wiederum mit gleichen Bezugsziffern gekennzeichnet.

Der Mitnehmerstift 72 ist hier durch die Gewindespindel 20 hindurchgeführt. Der Mitnehmerstift 72 ragt - wie besser in Figur 3 b zu erkennen - durch die schlitzförmigen Öffnungen 74 der Mitnehmergabel 70. Die Mitnehmergabel 70 ist hier, wie in Figur 2, als Hülse mit längslaufenden schlitzförmigen Öffnungen 74 ausgebildet. Wenn der Mitnehmerstift 72 nur einseitig aus der Spindel herausragt, genügt eine Schlitzförmige Öffnung 74. Grundsätzlich ist es nicht erforderlich, dass die Mitnehmergabel 70 eine weitgehend geschlossene Hülse bildet, da lediglich eine Anlagekante für den Mitnehmerstift 72 vorhanden sein muss. Die Mitnehmergabel 70 könnte daher auch konkret gabelförmig mit einer oder mehreren Stangen parallel zur Spindel 20 ausgebildet sein, wobei die Stangen einen Anschlag und eine Gleitschiene für den Mitnehmerstift bilden würden.

Bei Antrieb durch den Motor 30 dreht sich die Mitnehmergabel 70, wobei deren Welle 76 in einer Führung 78 gelagert ist. Die drehende Mitnehmergabel 70 nimmt den Mitnehmerstift 72 mit, der hierdurch die Drehung der Gabel oder Hülse auf die Bewegungsschraube 20 überträgt. Dabei gleitet der Mitnehmerstift 72 innerhalb der schlitzförmigen Öffnungen 74 nach vorne, weil gleichzeitig die Drehbewegung der Bewegungsschraube 20 durch die zum Gehäuse 10 ortsfeste Mutter 22 in eine Vorwärtsbewegung umgesetzt wird.

Die Enden des Mitnehmerstiftes 72 ragen durch die schlitzförmigen Öffnungen 74 bis in einen ringförmigen Schieber 80, der außerhalb der Hülse 70 sitzt und auf dieser gleitet. Der Schieber 80 wird durch den Mitnehmerstift 72 in Längsrichtung mitgenommen. Die Führung 82 verhindert, dass sich der Schieber mit der Hülse drehen kann. Der Schieber 80 kann sich maximal zwischen einer Position, die durch den Anschlag des Mitnehmerstiftes 72 am Ende der schlitzförmigen Öffnung 74 bestimmt wird, bis zu einem Anschlag 84 an der Mutter 22 bewegen. Diese Bewegung und die zugehörige Wegstrecke korrespondieren mit dem Weg des Spritzenkolbens 54.

Per Sensor, der die Bewegung des Schiebers 80 und dessen Position misst (Photosensor, nicht dargestellt), kann die vom Schieber zurückgelegte Wegstrecke und damit die Position des Spritzenkolbens 54 jederzeit genau bestimmt werden.

### Bezugszeichenliste

- 10: Gehäuse
- 20: Bewegungsschraube
- 22: Mutter
- 30: Motor
- 32: Gleitschiene
- 34: Schlitten
- 36: (Motor-)Welle
- 40: Einspannvorrichtung
- 50: Spritze
- 52: Spritzenkörper
- 54: (Spritzen-)Kolben
- 56: Kolbenstopp
- 60: Übertragung
- 70: Mitnehmergabel
- 72: Mitnehmerstift
- 74: Schlitz/schlitzförmige Öffnung
- 76: Welle der Mitnehmergabbel 70
- 78: Führung der Welle 76
- 80: Schieber
- 82: Führung für Schieber 80
- 84: Anschlag für Schieber 80

## Patentansprüche

1. Vorrichtung zum Antreiben eines Kolbens (54) einer Infusions- oder Injektionsspritze (50), wobei mit dem Kolben (54) der Spritze (50) unmittelbar oder über einen Adapter eine Bewegungsschraube (20) verbunden ist, wobei die schraubende Bewegung der Bewegungsschraube (20) auf den Kolben (54) übertragen wird, und dass die Bewegungsschraube (20) mit der Welle (36) eines kontinuierlich arbeitenden Motors (30) direkt, durch einen Überträger (60) oder Untersetzer (60) verbunden ist und eine Mutter (22) der Bewegungsschraube (20) an einem Gehäuseteil (10) der Vorrichtung befestigt ist.

2. Vorrichtung nach Anspruch 1, **dadurch gekennzeichnet, dass** die Vorrichtung eine Einspannvorrichtung (40) für die Spritze (50) umfasst.

3. Vorrichtung nach einem der Ansprüche 1 oder 2, **dadurch gekennzeichnet, dass** die Gewindespindel (20) der Bewegungsschraube formschlüssig mit dem Kolben (54) oder dem Adapter verbunden insbesondere verschraubt ist.

4. Vorrichtung nach einem der Ansprüche 1 bis 3, **dadurch gekennzeichnet, dass** der Motor (30) auf einem Schlitten (34) der Spindel (20) nachgeführt wird.

5. Vorrichtung nach einem der Ansprüche 1 bis 3, **dadurch gekennzeichnet, dass** der Motor (30) ortsfest angeordnet ist und die Motorwelle (36) mit einer Mitnehmergabel (70) verbunden ist, die an einem an der Bewegungsschraube (20) befestigten Mitnehmerstift (72) angreift.

6. Vorrichtung nach Anspruch 5, **dadurch gekennzeichnet, dass** die Mitnehmergabel (70) durch eine Hülse mit schlitzförmiger Öffnung (74) gebildet wird.

## Claims

1. Device for driving a piston (54) of an infusion or injection syringe (50), wherein a lead screw (20) is connected to the piston (54) of the syringe (50) directly or via an adapter, wherein the screwing movement of the lead screw (20) is transmitted to the piston (54), and that the lead screw (20) is connected to the shaft (36) of a continuously operating motor (30) directly, via a transmission (60) or reduction gear (60), and a nut (22) of the lead screw (20) is secured on a housing part (10) of the device.

2. Device according to Claim 1, **characterized in that** the device comprises a clamping device (40) for the syringe (50).

3. Device according to either of Claims 1 and 2, **characterized in that** the threaded spindle (20) of the lead screw is connected with form-fit engagement to the piston (54) or to the adapter, in particular screwed thereon.

4. Device according to one of Claims 1 to 3, **characterized in that** the motor (30) is guided along on a carriage (34) of the spindle (20).

5. Device according to one of Claims 1 to 3, **characterized in that** the motor (30) is arranged in a fixed position, and the motor shaft (36) is connected to a driver fork (70), which engages on a driver pin (72) secured on the lead screw (20).

6. Device according to Claim 5, **characterized in that** the driver fork (70) is formed by a sleeve with a slit-shaped opening (74).

## Revendications

1. Dispositif d'entraînement d'un piston (54) d'une seringue d'infusion ou d'injection (50), dans lequel une vis de déplacement (20) est reliée directement ou au moyen d'un adaptateur au piston (54) de la seringue (50), dans lequel le mouvement hélicoïdal de la vis de déplacement (20) est transmis au piston (54), et que la vis de déplacement (20) est reliée à l'arbre (36) d'un moteur (30) fonctionnant en continu directement, au moyen d'un organe de transmission (60) ou d'un réducteur (60) et un écrou (22) de la vis de déplacement (20) est fixé à une partie de boîtier (10) du dispositif.

2. Dispositif selon la revendication 1, **caractérisé en ce que** le dispositif comprend un dispositif de serrage (40) pour la seringue (50).

3. Dispositif selon une des revendications 1 ou 2, **caractérisé en ce que** la broche filetée (20) de la vis de déplacement est assemblée par emboîtement au piston (54) ou à l'adaptateur, en particulier vissée.

4. Dispositif selon l'une quelconque des revendications 1 à 3, **caractérisé en ce que** le moteur (30) est ajusté sur un chariot (34) de la broche (20).

5. Dispositif selon l'une quelconque des revendications 1 à 3, **caractérisé en ce que** le moteur (30) est disposé de façon fixe et l'arbre de moteur (36) est relié à une fourche d'entraîneur (70), qui s'applique sur une tige d'entraîneur (72) fixée sur la vis de déplacement (20).

6. Dispositif selon la revendication 5, **caractérisé en ce que** la fourche d'entraîneur (70) est formée par une douille avec une ouverture en forme de fente (74).
